# EUROPÄISCHE PATENTSCHRIFT

(11) **EP 3 853 864 B1**
(45) Veröffentlichungstag und Bekanntmachung des Hinweises auf die Patenterteilung: **30.10.2024**
(21) Anmeldenummer: 19769129.8
(22) Anmeldetag: 12.09.2019
(51) Int. Cl.: G16H 40/63

(54) **SICHERE EINGABE AUF EINEM STEUERUNGSBAUTEIL FUER EIN DIALYSEGERAET**
SECURE INPUT ON A CONTROL COMPONENT FOR A DIALYSIS DEVICE
ENTRÉE SÉCURISÉE SUR UN COMPOSANT DE COMMANDE D'UN DISPOSITIF DE DIALYSE

(30) Priorität: 19.09.2018 DE 102018123012
(43) Veröffentlichungstag der Anmeldung: 28.07.2021
(73) Patentinhaber: Fresenius Medical Care Deutschland GmbH, 61352 Bad Homburg (DE)
(72) Erfinder: MARTERSTOCK, Stefan Konrad, 97337 Dettelbach (DE)
(74) Vertreter: Schwarz, Claudia
(86) Internationale Anmeldenummer: PCT/EP2019/074349
(87) Internationale Veröffentlichungsnummer: WO 2020/058078

(56) Entgegenhaltungen:
- US-A- 5 792 204

## Beschreibung

Die Erfindung betrifft die sichere Eingabe von Steuerbefehlen zum Zwecke der Steuerung von medizinischen Geräten, insbesondere Dialysegeräten und ein diesbezügliches Schutzkonzept. Die Steuerbefehle werden auf einem Steuerungsbauteil eingegeben, das getrennt von dem zu steuernden Gerät bereitgestellt ist.

Dialysegeräte sind Blutbehandlungsgeräte, bei denen ein Fluid eines Patienten über eine Fluidleitung einer Fluidbehandlungskomponente zugeführt, durch die Fluidbehandlungskomponente behandelt und über die Fluidleitung, die in einen arteriellen und einen venösen Zweig aufgeteilt werden kann, wieder an den Patienten zurückgegeben wird. Beispiele für solche Blutbehandlungsgeräte sind insbesondere Hämodialysegeräte. Ein solches Blutbehandlungsgerät ist Gegenstand der DE 198 49 787 C1 der Anmelderin. wird. Verfahren der Hämodialyse, der Hämofiltration und der Hämodiafiltration werden in der Regel mit automatischen Hämodialysegeräten durchgeführt, wie sie beispielsweise von der Anmelderin vertrieben werden. Ein Plasmapheresegerät dient zur Ausführung einer Plasmapherese, einem Blutbehandlungsverfahren, bei dem das Patientenblut in das Blutplasma und seine korpuskularen Bestandteile (Zellen) aufgetrennt wird. Das abgetrennte Blutplasma wird gereinigt oder durch eine Substitutionslösung ersetzt und das gereinigte Blutplasma oder die Substitutionslösung dem Patienten zurückgegeben. Peritonealdialysegeräte dienen zur Durchführung einer Peritonealdialyse, bei der die Bauchhöhle eines Patienten über einen durch die Bauchdecke geführten Katheter mit einer Dialyseflüssigkeit befüllt wird, die ein Konzentrationsgefälle von Blutsubstanzen wie Elektrolyte (beispielsweise Natrium, Kalzium und Magnesium) gegenüber den körpereigenen Flüssigkeiten aufweist. Über das als Membran wirkende Bauchfell (Peritoneum) treten im Körper vorliegende Giftstoffe aus den im Bauchfell verlaufenden Blutgefäßen in die Bauchhöhle über. Nach einigen Stunden wird die sich in der Bauchhöhle des Patienten befindliche, nunmehr mit den aus dem Körper übergetretenen Giftstoffen versetzte Dialyseflüssigkeit ausgetauscht. Durch osmotische Vorgänge kann Wasser aus dem Blut des Patienten über das Bauchfell in die Dialyseflüssigkeit übertreten und den Patienten somit entwässern.

Diese vorstehend genannten und heute auf dem Markt befindlichen Dialysegeräte sind in der Regel autark, d.h. die Programmablauflogik zur Steuerung des Gerätes befindet sich auf dem Gerät selbst. Als Ein- und Ausgabeeinheit ist auf dem Dialysegerät mindestens eine Benutzeroberfläche ausgebildet.

Um die Flexibilität zu erhöhen, ist es wünschenswert, dass die Steuerung der Dialysegeräte auch auf entfernten Steuerungsgeräten erfolgen kann.

Bei medizinischen Geräten sind die Sicherheitsanforderungen jedoch deutlich erhöht im Vergleich zu anderen technischen Geräten. Deshalb muss auch bei einer entfernten Steuerung ein entsprechendes Sicherheitskonzept umgesetzt werden, um einerseits den Missbrauch von zu schützenden Daten (PHI-Daten, protected health data, wie z.B. Patientenbezogenen Daten) und andererseits die unbeabsichtigte und damit den Patienten möglicherweise gefährdende Einflussnahme auf die Steuerung des medizinischen Gerätes (z.B. durch Aufschaltung auf die Steuerleitung) sicher ausschließen zu können.

Aufgabe der vorliegenden Erfindung ist es deshalb, ein verbessertes Sicherheitskonzept für ein medizinisches Gerät oder eine Gruppe dieser Geräte bereitzustellen, das einerseits eine erhöhte Flexibilität der Eingabe von Steuerbefehlen ermöglicht und dabei andererseits auch den erhöhten Sicherheitsanforderungen durch strukturelle bauliche Maßnahmen entsprechen kann.

Diese Aufgabe wird erfindungsgemäß durch Geräte, Systeme und Verfahren gemäß den beiliegenden, einander nebengeordneten Patentansprüchen gelöst. Dokument US 5 792 204 A offenbart ein Verfahren zur Verifikation von Sprachbefehlen für medizinische Geräte, d.h. es wird im Wesentlichen festgestellt ob der vom Nutzer abgegebene Sprachbefehl auch vom System richtig erfasst wurde, ist dies der Fall, wird der Steuerungsbefehl nach Freigabe des Benutzers ausgeführt. Allerdings ist eine Verifikation mittels eines dem jeweiligen Steuerungsbefehl zugeordneten Bedienelements in diesem Dokument nicht vorgesehen.

Im Folgenden wird die Erfindung anhand der vorrichtungsgemäßen Aufgabenlösung, und somit anhand des Steuerungsgerätes beschrieben. Dabei erwähnte Merkmale, Vorteile oder alternative Ausführungsformen sind ebenso auch auf die anderen beanspruchten Gegenstände zu übertragen und umgekehrt. Mit anderen Worten können auch die verfahrensbasierten Ansprüche mit den Merkmalen weitergebildet sein, die in Zusammenhang mit dem Steuerungsgerät beschrieben und/oder beansprucht sind und umgekehrt. Die entsprechenden funktionalen Merkmale des Verfahrens werden dabei durch entsprechende gegenständliche Module, insbesondere durch elektronische Hardware-Module oder digitale Verarbeitungseinheiten (wie z.B. Mikrocontroller-Module oder Mikroprozessoreinheiten), des Systems bzw. des Produktes ausgebildet und umgekehrt. So kann z.B. das Erfassen eines Steuerbefehls durch eine entsprechende Eingabeschnittstelle ausgeführt werden, die z.B. als eine Schaltfläche auf einer (grafischen) Benutzeroberfläche ausgebildet sein kann.

Gemäß einem ersten Aspekt betrifft die Erfindung ein Steuerungsbauteil zur abgesicherten Erfassung von Steuerbefehlen, die zum Steuern von zumindest einem medizinischen Gerät, insbesondere einem Dialysegerät, dienen, mit:
- einer Eingabeschnittstelle zum Erfassen von zumindest einem Steuerbefehl;
- einer Ausgabeschnittstelle zur Ausgabe einer Nachricht, die eine Aufforderung zur Aktivierung zumindest eines Bedienelementes umfasst;
- einem Prozessor, der ausgebildet ist, einen Sicherungsalgorithmus zu implementieren, indem der Prozessor selbst prüft oder das zumindest eine Bedienelement mit einer Zusatzfunktion zum Prüfen beauftragt, ob die Aktivierung (z.B. mittels Erfassung eines Aktivierungssignals auf dem Bedienelement) auf dem zumindest einen Bedienelement erfasst wurde;
- zumindest einem Bedienelement, das vom Prozessor gesteuert wird und entsprechend seiner Beauftragung durch den Prozessor prüft, ob die Aktivierung erfasst wurde und wobei das zumindest eine Bedienelement bei Erfassung der Aktvierung dazu bestimmt ist, jeweils ein Bestätigungssignal an den Prozessor zu senden;
wobei der Prozessor des Steuerungsbauteils das Anwenden (bzw. Ausführen) des zumindest einen Steuerbefehls auf dem medizinischen Gerät nur dann veranlasst, wenn er das Betätigungssignal von dem zumindest einem Bedienelement empfangen hat.

In einer bevorzugten Ausführungsform der Erfindung ist es vorgesehen, dass der Steuerbefehl auf dem Steuerungsbauteil erfasst wird. Dann kann es z.B. weiterhin vorgesehen sein, dass der Steuerbefehl nur dann an das medizinische Gerät gesendet wird, wenn das Bestätigungssignal empfangen wurde.

In einer bevorzugten Ausführungsform der Erfindung sind die Eingabeschnittstelle und das zumindest eine Bedienelement technisch getrennte Bauteile. Die vorstehend genannten Elemente können als unterschiedliche Eingabekanäle implementiert sein (z.B. Touch- und/oder Hardkeys), die aber von einem Prozessor (vorzugsweise im Steuerungsbauteil) ausgelesen werden können Dies hat den Vorteil, dass die Eingabe nochmals auf einem anderen Medium (Element des Steuerungsbauteils) bestätigt werden kann. Damit kann die Eingabe sozusagen über einen zusätzlichen Eingabekanal abgesichert werden.

In einer weiteren, bevorzugten Ausführungsform der Erfindung ist das Steuerungsbauteil getrennt von dem medizinischen Gerät angeordnet bzw. als separates Gerät bereitgestellt. Dabei kommuniziert das Steuerungsbauteil über eine gesicherte Kommunikationsverbindung mit dem medizinischen Gerät. Alternativ kann das Steuerungsbauteil auch auf dem medizinischen Gerät ausgebildet sein.

In einer weiteren bevorzugten Ausführungsform der Erfindung sind die Ausgabeschnittstelle und die Eingabeschnittstelle in einem Bauteil fusioniert. Das Fusionsbauteil kann z.B. als - insbesondere grafische - Benutzeroberfläche ausgebildet sein.

In einer anderen bevorzugten Ausführungsform der Erfindung umfasst die Nachricht ein Rekapitulationselement, das den auf der Eingabeschnittstelle erfassten zumindest einen Steuerbefehl (oder die Folge davon) wiedergibt. Das Rekapitulationselement gibt die Steuerbefehle vorzugsweise in einem anderen Format wieder, z.B. falls akustisch eingegeben können sie textuell bzw. schriftbildliche im Rekapitulationselement wiedergegeben werden. Falls sie in einem anderen Beispiel durch eine Menüauswahl eingegeben wurden, können sie textuell wiedergegeben werden oder umgekehrt. Damit kann die Bestätigungssicherheit und damit auch die Qualität des gesamten Verfahrens verbessert werden, da Fehler bei einer wiederholten Darstellung (insbesondere mit einem Formatwechsel) häufig erkannt werden.

Gemäß einem weiteren Aspekt wird die Aufgabe gelöst durch ein Schutzverfahren zum Betrieb eines Steuerungsbauteils zur abgesicherten Erfassung von Steuerbefehlen zum Steuern von zumindest einem medizinischen Gerät (z.B. Dialysegerät), wobei das Steuerungsbauteil getrennt von dem medizinischen Gerät bereitgestellt ist und über eine gesicherte Kommunikationsverbindung mit dem medizinischen Gerät kommuniziert, mit folgenden Verfahrensschritten:
- Erfassen zumindest eines Steuerbefehls;
- Erzeugen einer Nachricht, die eine Aufforderung zur Aktivierung zumindest eines Bedienelementes umfasst;
- Ausgeben der erzeugten Nachricht:
- Prüfen, ob eine Aktivierung auf dem zumindest einen Bedienelement erfasst wurde und bejahendenfalls:
- (Lokales bzw. internes) Vorbereiten des erfassten zumindest einen Steuerbefehls zur Anwendung auf dem medizinischen Gerät.

Gemäß einer bevorzugten Ausführungsform der Erfindung des Schutzverfahrens wird der Steuerbefehl als akustische Eingabe (Spracheingabe) und/oder als Gesteneingabe (für eine Gestensteuerung) und/oder als Eingabe auf einer Benutzerschnittstelle erfasst. Alternativ kann eine Menüauswahl als Eingabe dienen. Damit kann die Flexibilität der Eingabe gesteigert werden.

In einer weiteren bevorzugten Ausführungsform der Erfindung wird überwacht, ob eine Aktivierung auf dem zumindest einem Bedienelement bereits zeitlich vor dem Ausgeben der Nachricht erfasst wurde und bejahendenfalls: Erfolgt ein Ausgeben eines Warnhinweises. Damit wird der technische Vorteil erreicht, dass automatisch erkannt werden kann, wenn z.B. ein Bedienelement nicht mehr fehlerfrei funktioniert und sozusagen "daueraktiviert" ist, weil z.B. eine Taste klemmt.

In einer weiteren bevorzugten Ausführungsform der Erfindung wird das Erfassen des zumindest einen Steuerbefehls nicht auf dem Bedienelement ausgeführt wird (sondern auf einer anderen Baueinheit, insbesondere auf einen zweiten Kanal). Alternativ oder kumulativ wird die Aktivierung nicht auf einer Eingabeschnittstelle ausgeführt bzw. erfasst, auf der der zumindest eine Steuerbefehl erfasst wird. Damit kann die Sicherheit erhöht werden, indem eine separate Bauteilaktivierung bzw. -bedienung erforderlich, so dass das separate Bauteil als Referenz bzw. unabhängige Bestätigung und damit als Kontrolle dienen kann.

In einer weiteren bevorzugten Ausführungsform der Erfindung umfassen der zumindest eine Steuerbefehl und/oder die Nachricht einen Zeitstempel. Damit kann noch genauer und auch in zeitlicher Hinsicht eine Überprüfung zwischen Eingabe und Bestätigung ausgeführt werden.

In einer weiteren bevorzugten Ausführungsform der Erfindung wird die Aufforderung zur Aktivierung zumindest eines (bestimmten) Bedienelementes aus einer Gruppe von Bedienelementen dynamisch nach einem vorkonfigurierten Schema erzeugt, das in einem Speicher gespeichert ist. Das Schema kann konstant für alle zu bestätigende Steuerbefehle übereinstimmend hinterlegt sein. Alternativ kann es auch für jeden oder eine Gruppe von Steuerbefehlen unterschiedlich sein. So kann sich das Schema also auch von Steuerbefehl zu Steuerbefehl unterscheiden. Das Schema kann z.B. kennzeichnen, in welcher Form und/oder wann ein Bedienelement zu aktivieren ist: Beispielsweise kann konfiguriert sein, dass zuerst ein erstes Bedienelement und dann ein zweites Bedienelement aktiviert werden muss. Falls ein Bedienelement z.B. eine Schaltfläche auf einer GUI ist, dann kann ein einfaches oder zweifaches Anklicken gefordert werden. Diese Einstellungen des Schemas werden in einer Vorbereitungsphase des Schutzverfahrens konfiguriert.

In einer anderen, bevorzugten Ausführungsform der Erfindung ist die Aufforderung zur Aktivierung zumindest eines Bedienelementes eine Aufforderung zur Aktivierung von mehreren Bedienelementen in einer definierten Abfolge oder zur zeitgleichen Aktivierung. Damit kann die Sicherheit erhöht werden, indem nicht nur eine Aktivierungshandlung sondern mehrere in einer vordefinierten Reihenfolge auszuführen sind. Somit muss sowohl die Reihenfolge eingehalten als auch das Bedienelement auf korrekte Weise bedient werden. Dies bedeutet eine zweifache Absicherung.

In einer anderen, bevorzugten Ausführungsform der Erfindung ist das Schutzverfahren in einem geschützten Speicherbereich des Steuerungsbauteils implementiert.

Gemäß einem weiteren Aspekt wird die vorstehende Aufgabe weiterhin gelöst durch ein Computerprogramm für ein medizinisches Gerät und/oder ein Steuerungsbauteil, das in einen internen Speicher eines Prozessors geladen werden kann und Softwareroutinen umfasst, mit denen die Schritte des Verfahrens gemäß den vorstehenden Verfahrensansprüchen ausgeführt werden, wenn die Softwareroutinen auf dem Prozessor ausgeführt werden. Das Schutzverfahren und/oder der Sicherungsalgorithmus können somit vollständig automatisch, computer-implementiert und insbesondere ohne Benutzerinteraktion ausgeführt werden.

Gemäß einem weiteren Aspekt wird die vorstehende Aufgabe weiterhin gelöst durch ein Steuerungssystem zur abgesicherten Erfassung von Steuerbefehlen auf einem Steuerungsbauteil, das zum Steuern von zumindest einem medizinischen Gerät (z.B. einem Dialysegerät) ausgebildet ist. Das System ist ausgebildet mit:
- dem zumindest einem medizinischen Gerät;
- dem Steuerungsbauteil;
- zumindest einem Bedienelement;

wobei das Steuerungsbauteil getrennt von dem medizinischen Gerät angeordnet ist und über eine gesicherte Kommunikationsverbindung mit dem medizinischen Gerät kommuniziert und damit sozusagen als verteilt realisiertes System ausgebildet ist,
wobei auf dem Steuerungsbauteil oder auf dem medizinischen Gerät ein Steuerbefehl erfasst wird und eine Nachricht erzeugt wird, die eine Aufforderung zur Aktivierung des zumindest einen Bedienelementes umfasst;
und wobei auf dem Steuerungsbauteil oder auf dem medizinischen Gerät die erzeugte Nachricht ausgegeben wird;
und wobei auf dem oder unter Zuhilfenahme des zumindest einen Bedienelement(s) geprüft wird, ob eine Aktivierung erfasst wurde und wobei bejahendenfalls (und damit gilt der erfasste zumindest eine Steuerbefehl als bestätigt):
   Veranlassen, dass der erfasste zumindest eine Steuerbefehl auf dem medizinischen Gerät angewendet wird.

Das Schutzverfahren kann mehrere Abschnitte umfassen, die auf unterschiedlichen computerbasierten Entitäten ausgeführt werden. So kann ein erster Abschnitt des Schutzverfahrens auf dem Steuerungsbauteil und ein zweiter Abschnitt auf dem medizinischen Gerät ausgeführt werden und/oder auf einer separaten Instanz. Es ist auch möglich, dass alle Verfahrensschritte des Schutzsystems auf dem Steuerungsbauteil ausgeführt werden. Alternativ ist es möglich, dass einige Verfahrensschritte, insbesondere die Eingabe des zumindest einen Steuerbefehls und/oder die Auswertung komplett durch das medizinische Gerät erfolgt.

Im Folgenden werden die Begrifflichkeiten der Anmeldung näher definiert.

Das Steuerungsbauteil ist ein elektronisches Endgerät mit einem Prozessor oder Microprozessor, der einen Sicherungsalgorithmus zur Absicherung einer Eingabe (z.B. ein Steuerbefehl als Benutzereingabe eines Anwenders) zur Steuerung des medizinischen Gerätes ausführt. Das Steuerungsbauteil kann direkt oder indirekt mit einer Benutzerschnittstelle ausgebildet sein. "Indirekt" meint in diesem Zusammenhang, dass die Benutzerschnittstelle auch auf einem externen Gerät ausgebildet sein kann (z.B. Mobilfunkgerät), das über eine entsprechende Netzwerkverbindung (z.B. WLAN) oder Schnittstelle mit dem Steuerungsgerät in Datenaustausch steht. Das Steuerungsbauteil steht darüber hinaus mit dem medizinischen Gerät in Datenverbindung. Vorzugsweise ist diese abgesichert. Das Steuerungsbauteil kann als Mobilfunkgerät oder als Tablet, Smartphone oder mobiles Endgerät ausgebildet sein. In einer Variante kann das Steuerungsbauteil als Server ausgebildet sein. Das Steuerungsbauteil kann zur (zentralen) Steuerung einer Mehrzahl von medizinischen Geräten ausgebildet sein.

Das Steuerungsbauteil wird bspw. durch einen Prozessor gesteuert. Die im Prozessor implementierte Software steuert insbesondere die Bedienelemente des Steuerungsbauteils. Erfindungsgemäß modifiziert der Sicherungsalgorithmus diese Software, um alle oder ausgewählte Bedienelemente mit einer zusätzlichen Funktionalität zu beauftragen. Dazu wird ein Bedienelement nach einem vorkonfigurierbaren Schema bestimmt, auf dem eine Benutzeraktion ausgeführt werden muss, um den eingegebenen und rekapitulierend angezeigten Steuerbefehl zu bestätigen. Dies kann z.B. mittels Umlegen eines Schalters oder mittels Anklicken einer Schaltfläche auf einer Benutzeroberfläche oder mittels Drückens oder Betätigens einer Taste z.B. Lauter und Leiser Taste an einem Tablet oder Smartphone ausgeführt werden. Sobald die vorgesehene Aktivierung des Bedienelementes erfasst werden konnte, sendet das Bedienelement ein Bestätigungssignal an den Prozessor, der daraufhin den jeweiligen Steuerbefehl als bestätigt kennzeichnet und die Ausführung des Steuerbefehls auf dem medizinischen Gerät veranlasst. Dies kann mitunter durch Versenden des Steuerbefehls an das medizinische Gerät erfolgen.

Es wird eine gesicherte Kommunikationsverbindung zwischen Steuerungsbauteil und dem zu steuernden medizinischen Gerät bereitgestellt. Die Eingaben, die auf dem Steuerungsbauteil als Steuerbefehle erfasst werden, werden später zur Steuerung des medizinischen Gerätes verwendet und müssen deshalb zusätzlich abgesichert werden. Dies wird erreicht, indem jede Eingabe über ein jeweils unterschiedliches (anderes) Bedienelement des Steuerungsbauteils bestätigt werden muss. Die Auswahl des zu betätigenden Bedienelementes erfolgt nach einem vordefinierten Schema, das über einen Sicherungsalgorithmus umgesetzt wird. Der Sicherungsalgorithmus ist im Prozessor des Steuerungsbauteils implementiert und kann in einem (vorzugsweise) gesicherten Speicherbereich gespeichert sein. Die gesicherte Kommunikationsverbindung kann somit vorzugsweise zum bidirektionalen Datenaustausch ausgebildet sein.

Das medizinische Gerät kann ein elektronisches Gerät sein, das medizinische Funktionen bereitstellt. Es kann sich insbesondere um ein Dialysegerät oder um ein anderes Blutbehandlungsgerät handeln. Das medizinische Gerät verfügt über einen Prozessor, z.B. einen digitalen Schaltkreis, der Steueraufgaben übernimmt.

Der Steuerbefehl dient zur Steuerung des medizinischen Gerätes und wird lokal auf dem Gerät ausgeführt und umgesetzt. Es kann sich z.B. um einen einzelnen Befehl (Pumpe aktivieren/deaktivieren) oder um eine Abfolge von Befehlen handeln (z.B. extrakorporales Blutbehandlungsmodul austauschen). Der Steuerbefehl wird in einem Format bereitgestellt, das vom Prozessor des medizinischen Gerätes umgesetzt werden kann.

Der Sicherungsalgorithmus ist vollständig computer-implementiert und wird automatisch ohne Benutzerinteraktionen ausgeführt. Der Sicherungsalgorithmus dient zur modifizierten Ansteuerung der Bedienelemente des Steuerungsbauteils (z.B. der "Lautsprecher lauter"-Taster wird mit der anderen oder zusätzlichen Funktion belegt, zu erfassen, ob eine Aktivierung bzw. Betätigung durch den Anwender stattgefunden hat oder nicht. Der Taster bzw. das Bedienelement wird somit ein Prüfelement zur Bestätigung eines bereits eingegebenen Steuerbefehls mittels Aktivierung einer Taste oder eines konfigurierbaren Schaltelementes oder Bauteils auf dem Steuerungsbauteil. Der Sicherungsalgorithmus kann im Prozessor des Steuerungsbauteils und/oder im Prozessor des Dialysegerätes implementiert sein. Im Vorfeld können in einer Konfigurationsphase bestimmte Konfigurationen durch den Anwender getroffen und hinterlegt werden, so z.B. das Schema zur Aktivierung der Bedienelemente. Dabei kann eingestellt werden, welche Bedienelemente (Schalter, Taster, Schaltflächen, Knöpfe etc.) mittels welcher Aktionen (einfaches oder wiederholtes Klicken, Drücken, Schalten, Drehen etc.) zur Bestätigung aktiviert werden müssen. Um die Sicherheit zu erhöhten, kann es konfiguriert werden, dass nicht nur ein Bestätigungselement z.B. (Lautsprechertaste) aktiviert werden muss, sondern eine Menge von Bestätigungselementen in einer konfigurierbaren Reihenfolge.

Das Bestätigungssignal ist ein elektronisches Signal und kann binär sein (den Zustand 0 oder 1 annehmen, um eine erfolgreiche oder fehlerhafte Bestätigung des Bedienelementes zu signalisieren) oder eine Sequenz von Bits sein (kodiert). Das Bestätigungssignal wird von dem jeweiligen Bedienelement an den Prozessor des jeweiligen Gerätes (Steuerungsbauteil oder medizinisches Gerät) gesendet.

Die Nachricht ist elektronisch und umfasst zumindest einen digitalen Datensatz. Die Nachricht enthält zumindest eine Aufforderung zur Aktivierung des zumindest einen Bedienelementes. Die Nachricht richtet sich an den Anwender und wird auf der Ausgabeeinheit (z.B. GUI) ausgegeben. Die Nachricht kann z.B. den textuellen oder sprach-basierten Hinweis umfassen: "Bitte betätigen Sie jetzt das Bedienelement des Gerätes XY an der Position xyz auf die beschriebene Weise". Dazu kann ein bildlicher Hinweis ausgegeben werden, wo sich das Bedienelement befindet und/oder eine simulierte Darstellung, wie es zu aktivieren ist. In einer bevorzugten Ausführungsform der Erfindung umfasst die Nachricht ein Rekapitulationselement. Dies kann z.B. ein Datenfeld sein, um dem Anwender den auf der Eingabeschnittstelle erfassten Steuerbefehl nochmals wiederholt als Sprach- oder Textformbasierte Rekapitulation zur Bestätigung anzuzeigen. Zusätzlich kann dem Anwender ein Bestätigungsfeld bereitgestellt werden, über das er die Eingabe seines Steuerbefehls bestätigen oder widerrufen kann. Nach einem Widerruf wird das Schutzverfahren unterbrochen. Nach einer Bestätigung wird es weiter ausgeführt. Es ist auch denkbar, dass das Bestätigungssignal durch Spracheingabe vom Bediener abgesetzt werden kann.

Das Prüfen (ob das vorgesehene Bedienelement auf vorgesehene Weise aktiviert worden ist) erfolgt vorzugsweise kontinuierlich und/oder mehrfach sobald die erste Eingabe möglich ist und dann ereignisbasiert, z.B. sobald eine Aktivierung erfasst wird oder sobald eine Nachricht erhalten wird. Damit wird erreicht, dass die Funktionalität des jeweiligen Bedienelementes überwacht werden kann. Wird nämlich bereits vor der Aufforderung zur Aktivierung des Bedienelementes ein Bestätigungssignal auf diesem erfasst, kann davon ausgegangen werden, dass es fehlerhaft ist (Taste klemmt) und ein entsprechender Warnhinweis kann ausgegeben werden.

Das Schutzverfahren und/oder der Sicherungsalgorithmus können als Computerprogramm bereitgestellt werden, insbesondere als Microprozessorprogramm.

Eine weitere Aufgabenlösung besteht somit in einem Computerprogrammprodukt, das in einen Speicher eines Computers oder eines elektronischen oder medizintechnischen Gerätes geladen oder ladbar ist mit einem Computerprogramm zur Durchführung des oben näher beschriebenen Schutzverfahrens, wenn das Computerprogramm auf dem Computer oder dem elektronischen oder medizintechnischen Gerät ausgeführt wird.

Eine weitere Aufgabenlösung sieht ein Computerprogramm vor zur Durchführung aller Verfahrensschritte des oben näher beschriebenen Verfahrens, wenn das Computerprogramm auf einem Computer, einem elektronischen oder medizintechnischen Gerät ausgeführt wird. Dabei ist es auch möglich, dass das Computerprogramm auf einem für den Computer oder das elektronische oder medizintechnische Gerät lesbaren Medium gespeichert ist.

In der folgenden detaillierten Figurenbeschreibung werden nicht einschränkend zu verstehende Ausführungsbeispiele mit deren Merkmalen und weiteren Vorteilen anhand der Zeichnung besprochen.

### Kurze Beschreibung der Figuren

Fig. 1 zeigt in einer schematischen Darstellung ein Dialysegerät, für das Steuerbefehle auf abgesicherte Weise auf einem getrennt bereitgestellten Steuerungsbauteil erfasst werden sollen gemäß einer vorteilhaften Ausführungsform der Erfindung.
Fig. 2 ist eine Alternative Implementierung des Ausführungsbeispiels, das in Fig. 1 dargestellt ist.
Fig. 3 ist ein exemplarisches Ablaufdiagramm von Verfahrensschritten eines Schutzverfahrens gemäß einer vorteilhaften Ausführungsform der Erfindung.
Fig. 4 zeigt ein exemplarisches Interaktionsdiagramm mit einem Datenaustausch von Signalen und Nachrichten zwischen dem Steuerungsbauteil und einem Dialysegerät.
Fig. 5 zeigt ein alternatives exemplarisches Interaktionsdiagramm mit einem Datenaustausch zwischen dem Steuerungsbauteil und einem Dialysegerät gemäß einer Variante der Erfindung.
Fig. 6 zeigt eine zu der in Fig. 1 dargestellten alternative Ausführung, bei der die Auswertelogik auf dem Dialysegerät ausgebildet ist.

### Detaillierte Beschreibung von Ausführungsbeispielen anhand der Figuren

Im Folgenden wird die Erfindung anhand von Ausführungsbeispielen im Zusammenhang mit den Figuren näher beschrieben. Die folgende Beschreibung der Ausführungsbeispiele bezieht sich auf ein Dialysegerät DG als Beispiel für ein medizinisches Gerät. In den bevorzugten Ausführungsform der Erfindung sind sowohl ein Steuerungsbauteil SB und ein Dialysegerät DG mit einem Prozessor MC ausgebildet. Um die Beschreibung möglichst übersichtlich zu halten, werden hierfür die gleichen Bezugszeichen verwendet, obwohl sich der Prozessor in unterschiedlichen Bauteilen befindet. Aus der Beschreibung geht allerdings eindeutig hervor, um welchen Prozessor es sich jeweils handelt.

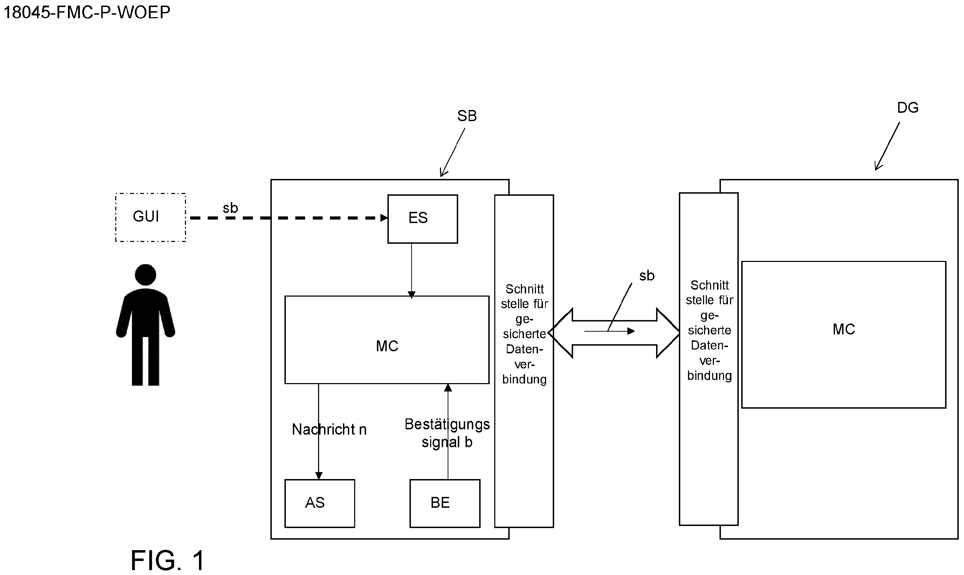

**Fig. 1** zeigt somit ein Dialysegerät DG als Beispiel für ein medizinisches Gerät, das von einem Steuerungsbauteil SB gesteuert werden soll. Der Anwender tätigt dazu Eingaben mit Steuerbefehlen sb auf dem Steuerungsbauteil SB. Dazu kann das Steuerungsbauteil SB mit einer Eingabeschnittstelle ES ausgebildet sein. Alternativ ist es auch möglich, die Eingabeschnittstelle ES, wie in Fig. 1 gestrichelt angedeutet, extern und außerhalb des Steuerungsbauteils SB auszubilden, z.B. als grafische Benutzeroberfläche GUI, die mit dem Steuerungsbauteil SB in Datenaustausch steht. Die auf der Eingabeschnittstelle erfassten Steuerbefehle sb werden an den lokalen Prozessor MC (also hier auf dem Steuerungsbauteil SB) weitergereicht, der das Erzeugen einer Nachricht n veranlasst, die auf einer Ausgabeschnittstelle AS ausgegeben wird. Die Nachricht n enthält eine Aufforderung zur Aktivierung eines Bedienelementes BE und kann ggf. noch weitere Hinweise und Datenfelder zur Benachrichtigung umfassen. Das jeweils in der Aufforderung indizierte Bedienelement BE wird überwacht. Insbesondere wird geprüft, ob eine Aktivierung erfolgt ist. Falls ja, wird ein Bestätigungssignal b an den Prozessor MC des Steuerungsbauteils SB übermittelt. Der Prozessor des Steuerungsbauteils SB ist nun in der Lage zu entscheiden, ob das Bedienelement, wie vom Sicherungsalgorithmus vorgesehen, bedient und aktiviert worden ist oder nicht. Falls ja (Bestätigungssignal b empfangen), kann er lokal Vorbereitungen treffen, damit der Steuerbefehl sb auf dem Dialysegerät DG umgesetzt und ausgeführt werden kann. Dazu kann er insbesondere das Versenden des Steuerbefehls sb über eine gesicherte Datenverbindung an das Dialysegerät DG veranlassen. Nach Empfang des Steuerbefehls sb auf dem Dialysegerät DG kann der Befehl dort ausgeführt werden.

In diesem Ausführungsbeispiel ist es vorgesehen, dass das Schutzverfahren vollständig auf dem Steuerungsbauteil SB ausgeführt wird. Der Anwender steuert somit das Dialysegerät entfernt (remote) vom Steuerungsbauteil SB aus.

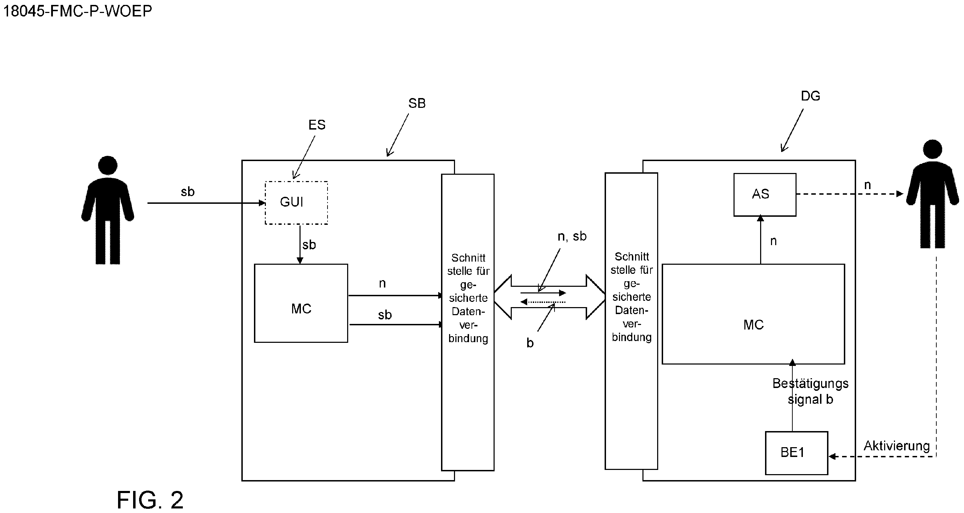

Eine alternative Variante ist in **Fig. 2** dargestellt. Hier kann z.B. ein 4-Augen-Prinzip zur Steuerung des Dialysegerätes DG umgesetzt werden. Ein erster Anwender bedient das Steuerungsbauteil SB und ein zweiter Anwender bedient das Dialysegerät DG. Ein Steuerbefehl sb wird vom ersten Anwender auf dem Steuerungsbauteil SB, z.B. auf einer Benutzeroberfläche GUI erfasst und an den Prozessor MC weitergeleitet, der in Antwort darauf die Nachricht n erzeugt und über die gesicherte Datenverbindung an das Dialysegerät DG sendet. Der dortige Prozessor MC des Dialysegerätes DG leitet die empfangene Nachricht n an die lokale Ausgabeschnittstelle AS zur Ausgabe (an den zweiten Anwender) weiter. Die Nachricht n enthält die Aufforderung zur Aktivierung eines Bedienelementes BE, das allerdings nun auf dem Dialysegerät DG angeordnet ist, so dass es vom zweiten Anwender oder auch vom selben Anwender bedient werden kann. Das Bedienelement BE wird vom Prozessor MC nach dem Sicherungsalgorithmus derart angesteuert, dass es bei korrekter Aktivierung (z.B. bei Erfassung eines Aktivierungssignals) das Bestätigungssignal b an den Prozessor MC des Dialysegerätes DG sendet.

In einer weiteren Variante erfolgt die Eingabe des Steuerbefehls sb am Steuerungsbauteil SB und die Ausgabe der Nachricht n wird ebenfalls auf dem Steuerungsbauteil SB ausgeführt, aber die Bestätigung der Eingabe wird am Dialysegerät DG vorgenommen (z.B. über einen dedizierten Bestätigungsschalter). Die Nachricht n kann auch als Sprachnachricht zur Ausgabe auf dem Steuerungsbauteil SB ausgebildet sein (z.B. laute sprachliche Wiedergabe der Benutzereingabe bzw. des Steuerbefehls mit einer Aufforderung zur Bestätigung).

In einer ersten Variante wird das Bestätigungssignal b (vom Dialysegerät DG) an das Steuerungsbauteil SB weitergeleitet, das in Antwort darauf, den (bestätigten) Steuerbefehl sb an den Prozessor MC des Dialysegerätes DG übermittelt.

In einer zweiten Variante wird vom Steuerungsbauteil SB nicht nur die lokal (oder in einer alternativen Ausführungsform der Erfindung auch auf dem Dialysegerät DG) erzeugte Nachricht n an das Dialysegerät DG zum Zwecke der Bestätigung gesendet, sondern zusätzlich auch der erfasste Steuerbefehl sb. In diesem Fall muss das Bestätigungssignal b vom Dialysegerät DG nicht an das Steuerungsbauteil SB gesendet werden, da das Dialysegerät bereits den Steuerbefehl sb kennt und diesen direkt ausführen kann. Falls kein Bestätigungssignal erfasst wurde, wird der vorhandene, aber unbestätigte Steuerbefehl sb gelöscht und nicht ausgeführt.

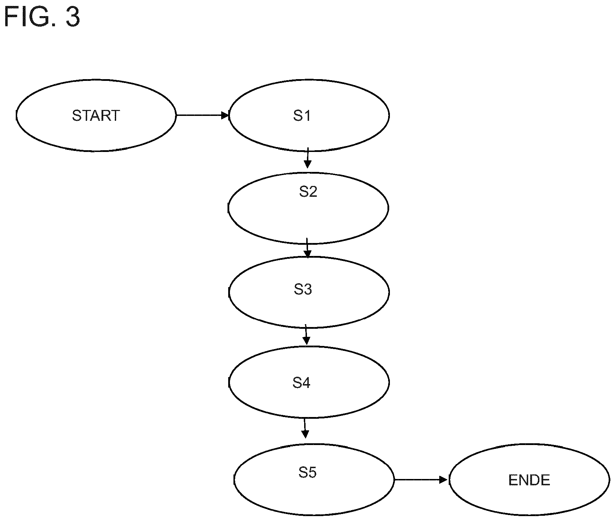

**Fig. 3** zeigt den Ablauf in einem Ausführungsbeispiel. Nach dem Start des Verfahrens wird in Schritt S1 der Steuerbefehl sb erfasst, vorzugsweise auf der Eingabeschnittstelle ES. In Schritt S2 wird die Nachricht n erzeugt. In Schritt S3 wird die erzeugte Nachricht n ausgegeben, vorzugsweise auf der Ausgabeschnittstelle AS. In Schritt S4 wird geprüft, ob das Bedienelement BE wie vorgesehen (gemäß Aufforderung) aktiviert worden ist. Dazu wird geprüft bzw. überwacht, ob ein Bestätigungssignal b auf dem Bedienelement BE erfasst und an den Prozessor MC übermittelt worden ist. In Schritt S5 wird der Steuerbefehl vorbereitet zur lokalen Ausführung auf dem Dialysegerät DG in Schritt S6.

Um die Sicherheitsanforderungen an das Steuerungsbauteil SB möglichst gering zu halten, ist es vorzugsweise vorgesehen, dass möglichst viele Schritte, insbesondere die Auswertung auf dem Dialysegerät DG selbst erfolgen. So ist es hilfreich, wenn ein erstes oder zweites Bedienelement BE1 auf dem Dialysegerät ausgebildet ist, wie oben in Zusammenhang mit Fig. 2 erläutert. Vorzugsweise ist deshalb das Steuerungsbauteil SB "nur" zur Datenein- und ausgabe ausgebildet, während die Auswertelogik mit der Prüfung, ob eine Aktivierung des Bedienelementes stattgefunden hat oder nicht, auf dem Dialysegerät selbst vorgenommen wird. Damit können die Sicherheitsstandards leichter eingehalten werden.

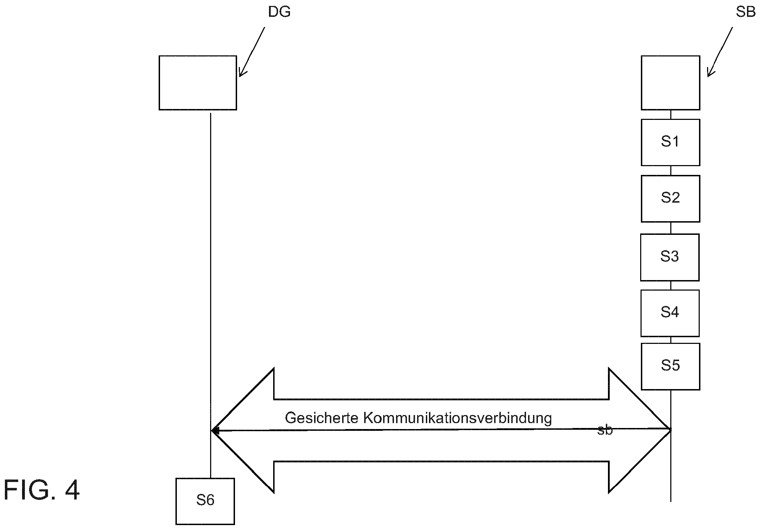

**Fig. 4** ist ein Interaktionsdiagramm zwischen dem Dialysegerät DG und dem Steuerungsbauteil SB. In der in Fig 4 gezeigten Variante werden alle Schritte des Schutzverfahrens auf dem Steuerungsbauteil SB ausgeführt. Erst nachdem eine erfolgreiche Bestätigung auf dem Steuerungsbauteil SB detektiert werden konnte, wird der Steuerbefehl sb an das Dialysegerät DG zur dortigen Ausführung gesendet. Andernfalls wird der

Steuerbefehl nicht umgesetzt und muss ggf. wiederholt eingegeben werden. Die Interaktionen in Fig. 4 entsprechen dem strukturellen Aufbau des Schutzsystems, das vorstehend unter Bezugnahme auf Fig. 1 beschrieben worden ist.

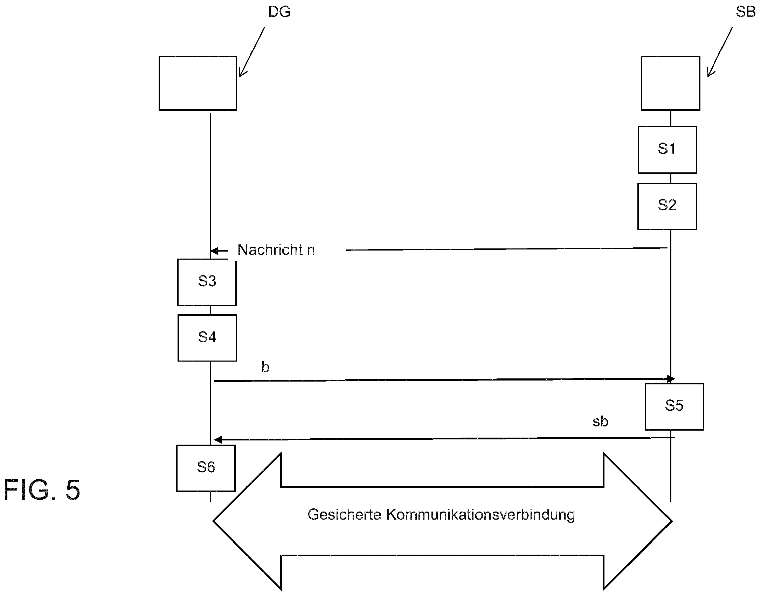

In **Fig. 5** ist eine Variante zu dem in Fig. 4 beschriebenen Ablauf gezeigt, die insofern dem strukturellen Aufbau entspricht, der vorstehend unter Bezugnahme auf Fig. 2 beschrieben worden ist. Das Schutzsystem ist hier als verteiltes System ausgebildet und das Schutzverfahren wird verteilt ausgeführt. Ein erster Abschnitt wird auf dem Dialysegerät DG und ein zweiter Abschnitt wird auf dem Steuerungsbauteil SB ausgeführt. Wie in Fig. 5 gezeigt, werden das Erfassen S1 und das Erzeugen der Nachricht n in Schritt S2 auf dem Steuerungsbauteil SB ausgeführt. Die Nachricht n wird an das Dialysegerät DG übermittelt. Vorzugsweise erfolgt dies auch über den gesicherten Datenkanal. In Antwort darauf, wird die Nachricht n dann in Schritt S3 ausgegeben und in Schritt S4 wird die Aktivierung des Bedienelementes BE geprüft. Falls ein Bestätigungssignal b erfasst wurde und das Prüfen somit ein erfolgreiches Prüfergebnis ermittelt hat, kann das Bestätigungssignal b in einer Variante an das Steuerungsbauteil SB übermittelt werden, das in Antwort darauf, den Steuerbefehl sb in Schritt S5 an das Dialysegerät DG übersendet, so dass dieses in Schritt 6 diesen lokal ausführen kann.

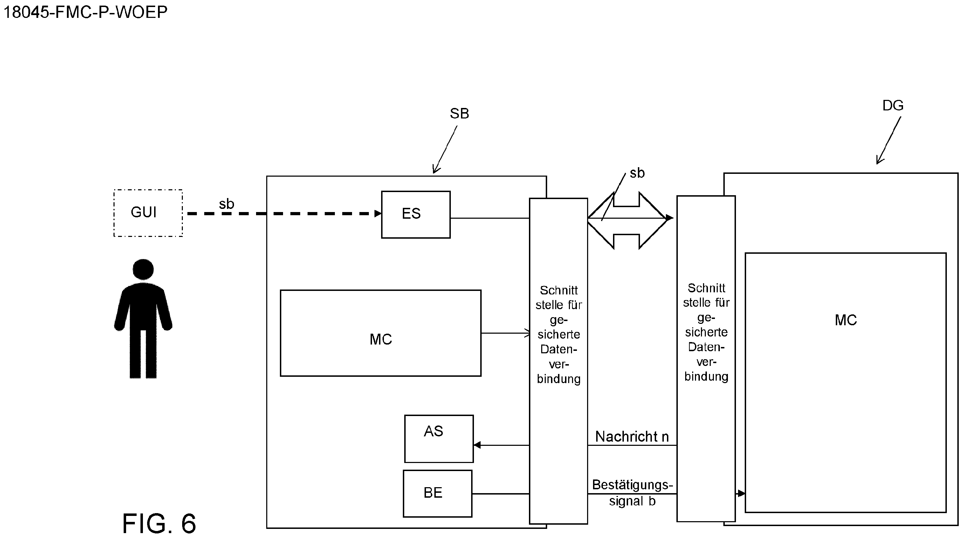

In **Fig. 6** ist eine Variante zu der in Fig. 1 gezeigten Ausführungsform der Erfindung dargestellt, bei der die Auswertelogik und der Sicherungsalgorithmus zumindest teilweise auf dem Dialysegerät DG ausgebildet sind. Der eingegebene Steuerbefehl sb wird an das Dialysegerät übertragen. Der Prozessor MC des Dialysegerätes DG setzt nun den erfassten Steuerbefehl sb in die Nachricht (Sprache oder Text) um und sendet diese an die Ausgabeschnittstelle AS des Steuerungsbauteils SB. Bei Aktivierung des Bedienelementes BE auf dem Steuerungsbauteils SB wird das Bestätigungssignal b an das Dialysegerät DG zurück gesendet. Damit gilt der vorher bereits übertragene Steuerbefehl sb als bestätigt. Mit anderen Worten wird der Steuerbefehl sb nur dann umgesetzt, wenn das Bestätigungssignal b auf dem Dialysegerät DG empfangen worden ist.

Durch die zusätzliche Überprüfung, ob ein Bedienelement auf vorkonfigurierte Weise zur Bestätigung des erfassten Steuerbefehls bedient worden ist oder nicht, können auch Eingaben und Steuerbefehle auf externen Geräten nochmals zusätzlich durch entsprechende Hardwarebaueile abgesichert werden. Ein Vorteil ist darin zu sehen, dass nicht neue Hardwarebauteile auf dem Endgerät (etwa in Form von neuen zusätzlichen Bedienelementen) bereitgestellt werden müssen, sondern dass die bereits vorhandenen Bedienelemente genutzt werden können und nur durch den Prozessor modifiziert angesteuert werden müssen.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren. Es liegt somit z.B. ebenso im Rahmen der Erfindung, nicht nur die Aktivierung eines Bedienelementes BE zu fordern, sondern die Aktivierung von mehreren Bedienelementen in einer vordefinierten Reihenfolge (z.B. erst Lautsprechertaster leise und dann Lautsprechertaster lauter). Für einen Fachmann ist es insbesondere offensichtlich, dass die Erfindung nicht nur für Dialysegeräte (z.B. für ein Hämodialysegerät oder ein Peritonealdialysegerät) angewendet werden kann, sondern auch für andere medizinische Geräte, die in abgesicherter Weise über ein entferntes Steuerungsbauteil SB gesteuert werden müssen.

Des Weiteren können die Bauteile des medizinischen Gerätes DG und des Steuerungsbauteils SB, wie z.B. die Benutzerschnittstelle GUI etc. auf mehrere physikalische Produkte verteilt realisiert werden.

Der Schutzbereich der vorliegenden Erfindung ist durch die Ansprüche gegeben und wird durch die in der Beschreibung erläuterten oder den Figuren gezeigten Merkmale nicht beschränkt.

## Patentansprüche

1. Steuerungsbauteil (SB) zur abgesicherten Erfassung von Steuerbefehlen zum Steuern von zumindest einem medizinischen Gerät (DG), insbesondere einem Dialysegerät, mit:
- einer Eingabeschnittstelle (ES) zum Erfassen von zumindest einem Steuerbefehl (sb);
- einer Ausgabeschnittstelle (AS) zur Ausgabe einer Nachricht (n), die eine Aufforderung zur Aktivierung zumindest eines Bedienelementes (BE) umfasst;
- einem Prozessor (MC), der ausgebildet ist, einen Sicherungsalgorithmus zu implementieren, indem der Prozessor (MC) das zumindest eine Bedienelement (BE) mit einer Zusatzfunktion zum Prüfen beauftragt, ob seine Aktivierung erfasst wurde;
- zumindest einem Bedienelement (BE), das vom Prozessor (MC) gesteuert wird und demzufolge prüft, ob die Aktivierung erfasst wurde und wobei das zumindest eine Bedienelement (BE) bei Erfassung der Aktvierung dazu bestimmt ist, jeweils ein Bestätigungssignal (b) an den Prozessor (MC) zu senden;
wobei der Prozessor (MC) das Anwenden des zumindest einen Steuerbefehls (sb) auf dem medizinischen Gerät (DG) nur dann veranlasst, wenn er das Betätigungssignal (b) von dem zumindest einem Bedienelement (BE) empfangen hat.

2. Steuerungsbauteil (SB) nach Anspruch 1, bei dem die Eingabeschnittstelle (ES) und das zumindest eine Bedienelement (BE) getrennte Bauteile sind.

3. Steuerungsbauteil (SB) nach zumindest einem der vorangehenden Ansprüche, bei dem das Steuerungsbauteil (SB) getrennt von dem medizinischen Gerät (DG) angeordnet ist und über eine gesicherte Kommunikationsverbindung mit dem medizinischen Gerät (DG) kommuniziert.

4. Steuerungsbauteil (SB) nach zumindest einem der vorangehenden Ansprüche, bei dem die Ausgabeschnittstelle (AS) mit der Eingabeschnittstelle (ES) in einem Bauteil fusioniert ist.

5. Steuerungsbauteil (SB) nach zumindest einem der vorangehenden Ansprüche, bei dem die Nachricht (n) ein Rekapitulationselement umfasst, das die auf der Eingabeschnittstelle (ES) erfassten Steuerbefehle (sb) wiedergibt.

6. Schutzverfahren zum Betrieb eines Steuerungsbauteils (SB) zur abgesicherten Erfassung von Steuerbefehlen (sb) zum Steuern von zumindest einem medizinischen Gerät (DG), insbesondere einem Dialysegerät, wobei das Steuerungsbauteil (SB) getrennt von dem medizinischen Gerät (DG) angeordnet ist und über eine gesicherte Kommunikationsverbindung mit dem medizinischen Gerät (DG) kommuniziert, mit folgenden Verfahrensschritten:
- Erfassen (S1) zumindest eines Steuerbefehls (sb);
- Erzeugen (S2) einer Nachricht (n), die eine Aufforderung zur Aktivierung zumindest eines Bedienelementes (BE) umfasst;
- Ausgeben (S3) der Nachricht (n);
- Prüfen (S4), ob eine Aktivierung auf dem zumindest einen Bedienelement (BE) erfasst wurde und bejahendenfalls:
- Vorbereiten (S5) des erfassten zumindest einen Steuerbefehls (sb) zur Ausführung (S6) auf dem medizinischen Gerät (DG).

7. Schutzverfahren nach dem vorstehenden Verfahrensanspruch, bei dem der zumindest ein Steuerbefehl (sb) als akustische Eingabe und/oder als Eingabe auf einer Benutzerschnittstelle erfasst wird.

8. Schutzverfahren nach zumindest einem der vorangehenden Verfahrensansprüche, bei dem das Prüfen (S4) ausgeführt wird, indem erfasst wird, ob ein Bestätigungssignal (b) empfangen wurde.

9. Schutzverfahren nach zumindest einem der vorangehenden Verfahrensansprüche, bei dem überwacht wird, ob eine Aktivierung auf dem zumindest einem Bedienelement (BE) bereits zeitlich vor dem Ausgeben der Nachricht (n) erfasst wurde und bejahendenfalls: Ausgeben eines Warnhinweises.

10. Schutzverfahren nach zumindest einem der vorangehenden Verfahrensansprüche, bei dem das Erfassen des zumindest einen Steuerbefehls (sb) nicht auf dem Bedienelement (BE) ausgeführt wird und/oder bei dem die Aktivierung nicht auf derjenigen Eingabeschnittstelle (ES) erfasst wird, auf der der zumindest eine Steuerbefehl (sb) erfasst wird.

11. Schutzverfahren nach zumindest einem der vorangehenden Verfahrensansprüche, bei dem der zumindest ein Steuerbefehl (sb) und/oder die Nachricht (n) einen Zeitstempel umfassen.

12. Schutzverfahren nach zumindest einem der vorangehenden Verfahrensansprüche, bei dem die Aufforderung zur Aktivierung zumindest eines Bedienelementes (BE) aus einer Gruppe von Bedienelementen dynamisch nach einem vorkonfigurierten Schema erzeugt wird, das in einem Speicher gespeichert ist.

13. Schutzverfahren nach zumindest einem der vorangehenden Verfahrensansprüche, bei dem die Aufforderung zur Aktivierung zumindest eines Bedienelementes (BE) eine Aufforderung zur Aktivierung von mehreren Bedienelementen (BE) in einer definierten Abfolge oder zur zeitgleichen Aktivierung ist.

14. Schutzverfahren nach zumindest einem der vorangehenden Verfahrensansprüche, das in einem geschützten Speicherbereich des Steuerungsbauteils (SB) implementiert ist.

15. Computerprogramm für ein medizinisches Gerät (DG) und/oder ein Steuerungsbauteil (SB), das in einen internen Speicher eines Prozessors (MC) geladen werden kann und Softwareroutinen umfasst, mit denen die Schritte des Verfahrens gemäß den vorstehenden Verfahrensansprüchen ausgeführt werden, wenn die Softwareroutinen auf dem Prozessor (MC) ausgeführt werden.

16. Steuerungssystem zur abgesicherten Erfassung von Steuerbefehlen (sb) auf einem Steuerungsbauteil (SB), das zum Steuern von zumindest einem medizinischen Gerät (DG), insbesondere einem Dialysegerät, ausgebildet ist, mit:
- dem zumindest einem medizinischen Gerät (DG);
- dem Steuerungsbauteil (SB);
- zumindest einem Bedienelement (BE);
wobei das Steuerungsbauteil (SB) getrennt von dem medizinischen Gerät (DG) angeordnet ist und über eine gesicherte Kommunikationsverbindung mit dem medizinischen Gerät (DG) kommuniziert und
wobei auf dem Steuerungsbauteil (SB) oder auf dem medizinischen Gerät (DG) ein Steuerbefehl (sb) erfasst wird und eine Nachricht (n) erzeugt wird, die eine Aufforderung zur Aktivierung des zumindest einen Bedienelementes (BE) umfasst;
und wobei auf dem Steuerungsbauteil (SB) oder auf dem medizinischen Gerät (DG) die erzeugte Nachricht (n) ausgegeben wird;
und wobei auf dem zumindest einen Bedienelement (BE) geprüft wird, ob eine Aktivierung erfasst wurde und bejahendenfalls:
Bestätigung des erfassten zumindest einen Steuerbefehls (sb) zur Veranlassung einer Anwendung desselben auf dem medizinischen Gerät (DG).

## Claims

1. Control component (SB) for the secure acquisition of control commands for controlling at least one medical device (DG), in particular a dialysis device, with:
- an input interface (ES) for capturing at least one control command (sb);
- an output interface (AS) for outputting a message (n) which comprises a request to activate at least one operating element (BE);
- a processor (MC) which is designed to implement a security algorithm in that the processor (MC) instructs the at least one operating element (BE) with an additional function for checking whether its activation has been detected;
- at least one operating element (BE) which is controlled by the processor (MC) and consequently checks whether the activation has been detected, and wherein the at least one operating element (BE) is configured to send a confirmation signal (b) to the processor (MC) when the activation is detected;
wherein the processor (MC) initiates the application of the at least one control command (sb) to the medical device (DG) only when it has received the confirmation signal (b) from the at least one operating element (BE).

2. Control component (SB) according to claim 1, in which the input interface (ES) and the at least one operating element (BE) are separate components.

3. Control component (SB) according to at least one of the preceding claims, wherein the control component (SB) is arranged separately from the medical device (DG) and communicates with the medical device (DG) via a secure communication link.

4. Control component (SB) according to at least one of the preceding claims, in which the output interface (AS) is fused with the input interface (ES) in one component.

5. Control component (SB) according to at least one of the preceding claims, wherein the message (n) comprises a recapitulation element reproducing the control commands (sb) detected on the input interface (ES).

6. Protection method for operating a control component (SB) for the secure acquisition of control commands (sb) for controlling at least one medical device (DG), in particular a dialysis device, the control component (SB) being arranged separately from the medical device (DG) and communicating with the medical device (DG) via a secure communication link, having the following method steps:
- Detection (S1) of at least one control command (sb);
- Generating (S2) a message (n), which comprises a request to activate at least one operating element (BE);
- Output (S3) of the message (n);
- Checking (S4) whether an activation has been detected on at least one operating element (BE) and if so:
- Preparing (S5) the captured at least one control command (sb) for execution (S6) on the medical device (DG).

7. Protection method according to the preceding method claim, in which the at least one control command (sb) is detected as an acoustic input and/or as an input on a user interface.

8. Protection method according to at least one of the preceding method claims, wherein the checking (S4) is performed by detecting whether a confirmation signal (b) has been received.

9. Protection method according to at least one of the preceding method claims, in which it is monitored whether an activation on the at least one operating element (BE) has already been detected at a time before the message (n) is output and, if the answer is affirmative, a warning is output.

10. Protection method according to at least one of the preceding method claims, in which the detection of the at least one control command (sb) is not carried out on the operating element (BE) and/or in which the activation is not detected on the input interface (ES) on which the at least one control command (sb) is detected.

11. Protection method according to at least one of the preceding method claims, in which the at least one control command (sb) and/or the message (n) comprise a time stamp.

12. Protection method according to at least one of the preceding method claims, in which the request to activate at least one operating element (BE) from a group of operating elements is generated dynamically according to a preconfigured scheme which is stored in a memory.

13. Protection method according to at least one of the preceding method claims, in which the request for activation of at least one operating element (BE) is a request for activation of a plurality of operating elements (BE) in a defined sequence or for simultaneous activation.

14. Protection method according to at least one of the preceding method claims, which is implemented in a protected memory area of the control component (SB).

15. Computer program for a medical device (DG) and/or a control component (SB), which can be loaded into an internal memory of a processor (MC) and comprises software routines with which the steps of the method according to the preceding method claims are carried out when the software routines are executed on the processor (MC).

16. Control system for the secure acquisition of control commands (sb) on a control component (SB) which is designed to control at least one medical device (DG), in particular a dialysis device, having:
- at least one medical device (DG);
- the control component (SB);
- at least one operating element (BE);
wherein the control component (SB) is arranged separately from the medical device (DG) and communicates with the medical device (DG) via a secure communication link, and wherein a control command (sb) is detected on the control component (SB) or on the medical device (DG) and a message (n) is generated which comprises a request to activate the at least one operating element (BE);
and whereby the generated message (n) is output on the control component (SB) or on the medical device (DG);
and whereby a check is carried out on the at least one operating element (BE) to determine whether an activation has been detected and, if so;
Confirmation of the captured at least one control command (sb) to initiate an application of the same on the medical device (DG).

## Revendications

1. Composant de commande (SB) pour la saisie sécurisée d'instructions de commande pour la commande d'au moins un appareil médical (DG), en particulier un appareil de dialyse, comprenant :
- une interface d'entrée (ES) pour la saisie d'au moins une instruction de commande (sb) ;
- une interface de sortie (AS) pour émettre un message (n) qui comprend une invitation à activer au moins un élément de commande (BE) ;
- un processeur (MC) qui est conçu pour mettre en oeuvre un algorithme de sécurité, en ce que le processeur (MC) charge l'au moins un élément de commande (BE) d'une fonction supplémentaire pour vérifier si son activation a été détectée ;
- au moins un élément de commande (BE) qui est commandé par le processeur (MC) et qui vérifie par conséquent si l'activation a été détectée et dans lequel le au moins un élément de commande (BE) est destiné, lors de la détection de l'activation, à envoyer respectivement un signal de confirmation (b) au processeur (MC) ;
dans lequel le processeur (MC) provoque l'application de la au moins une instruction de commande (sb) sur le dispositif médical (DG) uniquement lorsqu'il a reçu le signal de confirmation (b) provenant du au moins un élément de commande (BE).

2. Composant de commande (SB) selon la revendication 1, dans lequel l'interface d'entrée (ES) et l'au moins un élément de commande (BE) sont des composants séparés.

3. Composant de commande (SB) selon au moins l'une des revendications précédentes, dans lequel le composant de commande (SB) est disposé séparément du dispositif médical (DG) et communique avec le dispositif médical (DG) via une liaison de communication sécurisée.

4. Composant de commande (SB) selon au moins l'une des revendications précédentes, dans lequel l'interface de sortie (AS) est fusionnée avec l'interface d'entrée (ES) dans un composant.

5. Composant de commande (SB) selon au moins l'une des revendications précédentes, dans lequel le message (n) comprend un élément de récapitulation qui reproduit les instructions de commande (sb) saisies sur l'interface d'entrée (ES).

6. Procédé de protection pour le fonctionnement d'un composant de commande (SB) pour la saisie sécurisée d'instructions de commande (sb) pour la commande d'au moins un appareil médical (DG), en particulier un appareil de dialyse, le composant de commande (SB) étant disposé séparément de l'appareil médical (DG) et communiquant avec l'appareil médical (DG) par l'intermédiaire d'une liaison de communication sécurisée, comprenant les étapes de procédé suivantes :
- la détection (S1) d'au moins une instruction de commande (sb) ;
- générer (S2) un message (n), qui comprend une demande d'activation d'au moins un élément de commande (BE) ;
- Édition (S3) du message (n) ;
- vérifier (S4) si une activation a été détectée sur l'au moins un élément de commande (BE) et, si c'est le cas, répondre par l'affirmative :
- préparer (S5) la au moins une instruction de commande (sb) détectée pour l'exécution (S6) sur le dispositif médical (DG).

7. Procédé de protection selon la revendication de procédé précédente, dans lequel ladite au moins une instruction de commande (sb) est détectée en tant qu'entrée acoustique et/ou en tant qu'entrée sur une interface utilisateur.

8. Procédé de protection selon au moins l'une des revendications de procédé précédentes, dans lequel le test (S4) est effectué en détectant si un signal d'accusé de réception (b) a été reçu.

9. Procédé de protection selon au moins l'une des revendications de procédé précédentes, dans lequel on surveille si une activation sur l'au moins un élément de commande (BE) a déjà été détectée dans le temps avant l'émission du message (n) et, dans l'affirmative : on émet un avertissement.

10. Procédé de protection selon au moins l'une des revendications de procédé précédentes, dans lequel la saisie de l'au moins une instruction de commande (sb) n'est pas effectuée sur l'élément de commande (BE) et/ou dans lequel l'activation n'est pas saisie sur l'interface d'entrée (ES) sur laquelle l'au moins une instruction de commande (sb) est saisie.

11. Procédé de protection selon au moins l'une des revendications de procédé précédentes, dans lequel ladite au moins une instruction de commande (sb) et/ou ledit message (n) comprennent un horodatage.

12. Procédé de protection selon au moins l'une des revendications de procédé précédentes, dans lequel la demande d'activation d'au moins un élément de commande (BE) parmi un groupe d'éléments de commande est générée dynamiquement selon un schéma préconfiguré qui est enregistré dans une mémoire.

13. Procédé de protection selon au moins l'une des revendications de procédé précédentes, dans lequel la demande d'activation d'au moins un élément de commande (BE) est une demande d'activation de plusieurs éléments de commande (BE) dans une séquence définie ou pour une activation simultanée.

14. Procédé de protection selon au moins l'une des revendications de procédé précédentes, qui est mis en oeuvre dans une zone de mémoire protégée du composant de commande (SB).

15. Programme informatique pour un dispositif médical (DG) et/ou un composant de commande (SB), qui peut être chargé dans une mémoire interne d'un processeur (MC) et qui comprend des routines logicielles avec lesquelles les étapes du procédé selon les revendications de procédé précédentes sont exécutées lorsque les routines logicielles sont exécutées sur le processeur (MC).

16. Système de commande pour la saisie sécurisée d'instructions de commande (sb) sur un composant de commande (SB), qui est conçu pour commander au moins un appareil médical (DG), en particulier un appareil de dialyse, comprenant :
- d'au moins un dispositif médical (DG) ;
- le composant de commande (SB) ;
- au moins un élément de commande (BE) ;
dans lequel le composant de commande (SB) est disposé séparément du dispositif médical (DG) et communique avec le dispositif médical (DG) par l'intermédiaire d'une liaison de communication sécurisée et
dans lequel, sur le composant de commande (SB) ou sur le dispositif médical (DG), une instruction de commande (sb) est détectée et un message (n) est généré, qui comprend une demande d'activation de l'au moins un élément de commande (BE) ;
et dans lequel le message (n) généré est émis sur le composant de commande (SB) ou sur l'appareil médical (DG) ;
et dans lequel on vérifie sur l'au moins un élément de commande (BE) si une activation a été détectée et dans l'affirmative :
la confirmation de l'au moins une instruction de commande (sb) détectée pour provoquer son application sur le dispositif médical (DG).
